# FASCICULE DE BREVET EUROPEEN

(11) **EP 0 403 629 B1**
(45) Date de publication et mention de la délivrance du brevet: **16.03.1994**
(21) Numéro de dépôt: 90900893.0
(22) Date de dépôt: 19.12.1989
(51) Int. Cl.: C12M 3/00

(54) **BIOREACTEUR ET DISPOSITIF POUR LA CULTURE DE CELLULES ANIMALES**
BIOREAKTOR UND VORRICHTUNG ZUR KULTIVIERUNG VON TIERISCHEN ZELLEN
BIOREACTEUR AND DEVICE FOR THE CULTURE OF ANIMAL CELLS

(30) Priorité: 20.12.1988 FR 8816851
(43) Date de publication de la demande: 27.12.1990
(73) Titulaire: COMMISSARIAT A L'ENERGIE ATOMIQUE, 75015 Paris Cédex 15 (FR)
(72) Inventeur: BESNAINOU, Bernard, F-13100 Aix-en-Provence (FR); PHILIPPE, Alain, F-84120 Pertuis (FR); LESSART, Pierre, F-04130 Volx (FR); ELLUARD, Marie-Paule, F-84120 Pertuis (FR)
(74) Mandataire: Signore, Robert
(86) Numéro de dépôt international: FR8900664
(87) Numéro de publication internationale: WO9006990

(56) Documents cités:
- WO-A-86/02379

## Description

La présente invention a pour objet un bioréacteur et un dispositif pour la culture en continu et à l'échelle industrielle de cellules animales. Elle s'applique à la production de toutes cellules animales et à la production de métabolites produits par les cellules comme par exemple les anticorps monoclonaux. Le bioréacteur et le dispositif de l'invention permettent la culture dans des conditions stériles de cellules animales telles que des hybridomes.

On connaît déjà divers bioréacteurs pouvant être utilisés en culture de cellules libres (non-adhérentes) tels que ceux décrits dans les documents EP-A-0 113 328, EP-A-0 112 155, US-A-4 661 455, EP-A-0 155 237 et EP-A-0 112 154. Dans tous ces bioréacteurs connus, on utilise des membranes imperméables à l'eau mais perméables aux gaz afin d'assurer le transfert d'oxygène nécessaire au développement de toute cellule animale. Lors de l'extrapolation en taille de ces réacteurs, le problème des surfaces de transfert se pose très vite : par exemple, il a été calculé que pour un réacteur type BRAUN, équipé d'une cuve agitée avec tubes de silicone pour le transfert d'oxygène, il faudrait 700 mètres de tuyaux pour un fermenteur de 75 l.

En outre, les quatre dernières références se rapportent à des réacteurs constitués de membranes planes présentant l'inconvénient de réaliser des transferts non homogènes.

Par ailleurs, il n'y a aucun moyen de régulation et de contrôle du colmatage de ces membranes par les cellules cultivées. En particulier, le premier document cité force le passage de la solution contenant les cellules à travers des membranes. Le résultat immédiat en est un transfert de substances bien plus important, mais un effet secondaire en est une augmentation de la pression de transfert et une compression de la couche de polarisation qui s'est formée sur les membranes, ce qui a pour conséquence une augmentation du risque de colmatage desdites membranes.

Enfin, ces appareils basés sur le confinement des cellules entre deux types de membranes différentes, les unes servant à apporter certains substrats aux cellules et les autres servant à l'extraction de certains produits ne possèdent aucun dispositif de régulation et de contrôle intermédiaire de la perte de charge et présentent donc des fractions de membrane mal utilisées, les pressions étant voisines de part et d'autre des membranes, dans certaines zones, ce qui entraîne un transfert faible. Ce transfert faible ne permet pas l'apport contrôlé et intensif des substrats nutritifs nécessaires.

Par ailleurs, le colmatage progressif des membranes entraîne une diminution simultanée des transferts de tous les substrats nourriciers, mais dans des proportions souvent différentes, ce qui gêne le contrôle des concentrations au cours du temps, empêchant le fonctionnement de ces réacteurs dans de bonnes conditions sur de longues périodes.

L'invention a justement pour but un bioréacteur et un dispositif pour la culture de cellules animales permettant de remédier aux différents inconvénients donnés ci-dessus.

L'invention a donc pour objet un bioréacteur pour la culture de cellules animales comportant :
- une chambre de culture cellulaire, formée par une virole et les parois en regard de deux enceintes, respectivement enceinte d'entrée et enceinte de sortie, renfermant à la fois tes cellules et le milieu de culture, chaque enceinte étant équipée d'une cloison interne partageant ladite enceinte en une première et une seconde chambres respectivement intérieure et extérieure ;
- au moins un premier tube minéral poreux reliant la chambre externe de l'enceinte d'entrée à la chambre interne de l'enceinte de sortie et traversant ladite chambre de culture pour alimenter le milieu de culture en milieu nourricier, ledit premier tube minéral poreux comportant sur sa face interne une première membrane filtrante microporeuse autorisant le passage des molécules du milieu nourricier de masse moléculaire moyenne, mais arrêtant les macromolécules de masse moléculaire élevée, et sur sa face externe une seconde membrane microfiltrante permettant le passage des molécules de faible masse moléculaire et jouant te rôle de barrière pour tes cellules et ta plupart des protéines présentes dans la chambre de culture cellulaire ;
- au moins un deuxième tube minéral poreux reliant la chambre interne de l'enceinte d'entrée à la chambre externe de l'enceinte de sortie en traversant la chambre de culture cellulaire dont il permet d'extraire les métabolites de faible masse moléculaire, ledit deuxième tube comportant sur ses faces externe et interne des membranes filtrantes identiques à celles du premier tube minéral poreux ;
- une conduite de liaison entre les deux chambres internes des enceintes d'entrée et de sortie, dont la perte de charge supplémentaire a pour conséquence un meilleur transfert de matière à travers les premiers et seconds tubes minéraux poreux, et qui en provoquant une circulation de même sens du milieu nourricier à travers lesdits premiers et seconds tubes minéraux poreux, permet d'améliorer l'homogénéité du bioréacteur.

Par molécules de masses moléculaires moyennes, il faut comprendre des molécules de masses moléculaires comprises entre 50 000 et 300 000 Daltons ; les molécules de masses moléculaires faibles ont donc des masses moléculaires inférieures à 50 000 Daltons et tes molécules de masses moléculaires élevées ont des masses moléculaires supérieures à 300 000 Daltons.

Selon l'invention, la première membrane filtrante sert aussi de barrière aux particules telles que les virus, les micro-organismes, les débris cellulaires et les gels.

Les métabolites de faible masse moléculaire sont en particulier les ions ammonium, le dioxyde de carbone ou le lactate.

Avantageusement, la conduite de liaison entre les deux chambres internes est équipée d'une vanne de réglage permettant d'ajuster et de contrôler la perte de charge dans ladite conduite et d'obtenir ainsi un transfert très sensiblement constant sur toute la longueur desdits premiers et seconds tubes minéraux poreux.

Le bioréacteur peut fonctionner en discontinu (milieu non renouvelé) ou en continu. Dans ce dernier cas, un perfectionnement du bioréacteur consiste à faire traverser la chambre de culture par au moins un troisième tube minéral poreux isolé par rapport aux deux enceintes et ayant la face extérieure recouverte d'une membrane filtrante microporeuse imperméable aux cellules, mais perméable aux macromolécules produites dans la chambre de culture cellulaire. Ces macromolécules peuvent ainsi être soutirées pendant le fonctionnement du bioréacteur par l'intermédiaire d'une conduite connectée à l'extrémité ouverte dudit troisième tube poreux.

Les membranes filtrantes microporeuses sont de préférence des membranes minérales et en particulier des membranes en Al₂O₃, TiO₂ ou ZrO₂ bien que d'autres matériaux minéraux tels que te carbone pyrolitique puissent être utilisés. Cependant, des membranes organiques stérilisables à ta vapeur, telles que les membranes en polyfluorure de vinylidène, peuvent également être utilisées. Les tubes poreux assurant le support de ces membranes sont réalisés préférentiellement en carbone poreux ou en alumine, bien que d'autres matériaux tels que les métaux ou les céramiques frittés ou tissés puissent être utilisés.

Afin de limiter le colmatage des membranes déposées sur les faces des premiers et seconds tubes poreux et obtenir ainsi des caractéristiques de transfert sensiblement constantes pendant toute la durée de la culture, des moyens sont prévus pour inverser le sens de passage du milieu nourricier à l'intérieur desdits premiers et seconds tubes poreux. Dans ces conditions, les sens de transfert des premiers et des seconds tubes par rapport à la chambre de culture cellulaire sont inversés, et l'enceinte d'entrée devient l'enceinte de sortie et réciproquement.

L'augmentation du débit de transfert des premiers tubes vers la chambre de culture cellulaire et de ladite chambre de culture vers les seconds tubes peut être obtenue en augmentant le débit d'alimentation du milieu nourricier et/ou en pulsant ledit débit d'alimentation, et/ou en diminuant l'ouverture de la vanne de réglage de la conduite de liaison entre les deux chambres intérieures des enceintes d'entrée et de sortie, ce qui provoque une augmentation de la pression du milieu nourricier dans le circuit en amont de la vanne.

L'homogénéité de la chambre de culture peut être améliorée par des moyens permettant une rotation alternative de 180° dudit bioréacteur autour d'un axe longitudinal parallèle aux tubes poreux.

L'invention a aussi pour objet un dispositif pour la culture en continu de cellules animales comprenant un bioréacteur tel que défini comme précédemment, connecté à un réacteur de contrôle et de préparation du milieu nourricier, via des conduites souples de préférence en silicone, et des moyens pour extraire en continu ou séquentiellement les substances produites par les cellules.

D'autres caractéristiques et avantages de l'invention ressortiront mieux de la description qui va suivre, donnée à titre illustratif et non limitatif, en référence aux dessins annexés, dans lesquels :
- la figure 1 représente un schéma d'ensemble d'un dispositif de culture de cellules animales conforme à l'invention,
- la figure 2 représente de façon plus détaillée et en coupe longitudinale te bioréacteur du dispositif de la figure 1, et,
- la figure 3 représente une partie agrandie de la figure 2, montrant tes différentes membranes filtrantes du bioréacteur.

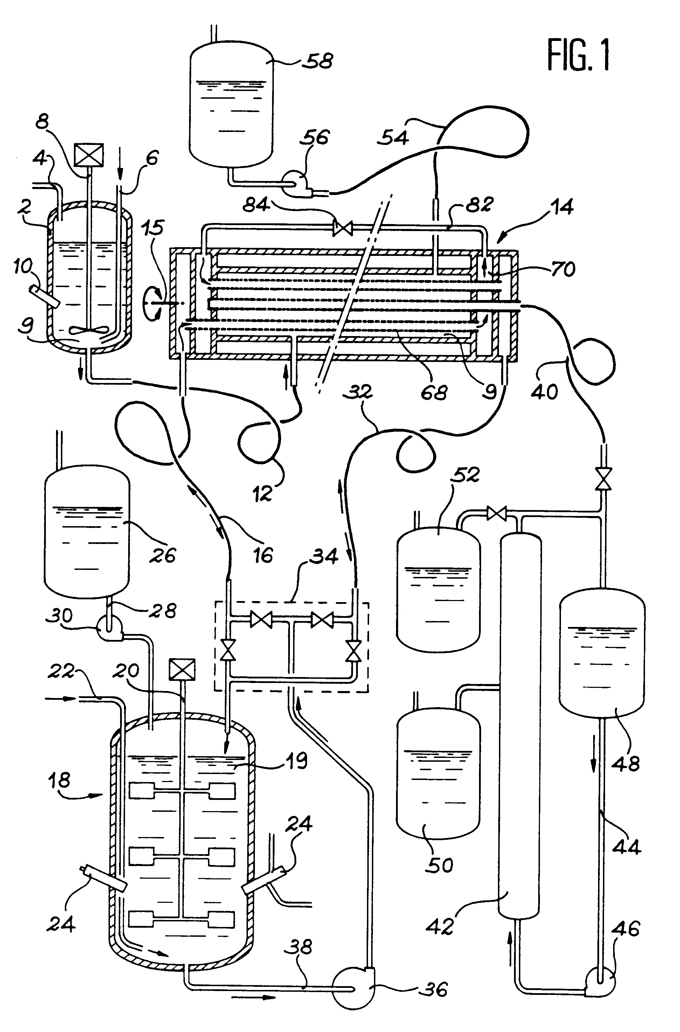

En référence à la figure 1, te dispositif pour la culture de cellules animales de l'invention comprend une cuve 2 destinée à la préculture des cellules animales, équipée d'une alimentation 4 en CO₂ et en air et d'une alimentation 6 en solution nutritive, le mélange gazeux et la solution nutritive étant nécessaires au développement des cellules animales contenues dans le milieu de culture 9. Un agitateur 8 assure une agitation lente du milieu de culture 9. Des capteurs 10 pour te contrôle de ce milieu, et en particulier du pH, de la pression en oxygène et de la pression en dioxyde, sont prévues. La cuve 2 et son équipement sont utilisés comme inoculum du bioréacteur.

La cuve 2 est reliée via une conduite souple 12 à un bioréacteur 14 dans lequel s'effectue la culture à proprement parler des cellules animales pour la production de métabolites par ces cellules. Le bioréacteur comporte un axe de symétrie longitudinal 15.

Le bioréacteur 14 est équipé d'une conduite d'amenée 16 du milieu nourricier de faible poids moléculaire (inférieur à 50 000 Daltons) nécessaire à la vie des cellules animales contenues dans le milieu de culture 9. Cette conduite d'amenée 16 est une conduite souple et est connectée à un réacteur auxiliaire 18 destiné à ta préparation du milieu nourricier et au contrôle de ce dernier.

La régulation du pH, de la pression en oxygène, de la pression en dioxyde de carbone, de la glutamine, du glucose et des acides aminés du milieu nourricier est effectuée dans ce réacteur auxiliaire 18. Le milieu nourricier porte la référence 19. Un système 20 permet son agitation. L'absence de protéines et de cellules dans le réacteur 18 permet l'utilisation d'une agitation brutale du milieu nourricier 19 (les contraintes de cisaillement ne sont pas limitantes) et d'un fort débit d'air et de dioxyde de carbone (pas de formation anormale de mousse) ; l'air et le CO₂ sont introduits via la conduite d'amenée 22 dans le réacteur 18.

Ce réacteur 18 est comme toutes les canalisations et autres réservoirs du dispositif stérilisable in situ à la vapeur.

Le réacteur 18 est équipé d'un dispositif 24 de prélèvements stériles du milieu nourricier 19 assurant un prélèvement continu ou séquenciel d'échantillons injectés automatiquement dans des analyseurs reliés à un système informatique qui contrôle ce milieu nourricier 19. Ce système permet l'injection à la demande de glucose, de glutamine ou d'acides aminés stockés dans un réservoir 26 connecté au réacteur 18 via une conduite 28 équipée d'une pompe 30.

Le système de stérilisation de l'ensemble du dispositif ainsi que le contrôle du milieu de culture et du milieu nourricier de façon automatique sont bien connus de l'homme de l'art et ne seront pas décrits plus en détail.

Le bioréacteur 14 est en outre équipé d'une conduite souple 32 de sortie du milieu nourricier 19 connectée au réacteur 18.

Un dispositif de vannes 34 permet d'inverser périodiquement le sens de circulation du milieu nourricier dans te bioréacteur 14 afin d'éviter son encrassement.

Une pompe 36, montée sur une conduite rigide 38 de sortie du milieu nourricier 19 du réacteur 18 et connectée au dispositif 34, assure la mise en circulation du milieu nourricier.

Les métabolites produits par les cellules dans le bioréacteur 14 sont extraits de celui-ci via une conduite souple 40 connectée à un système 42 d'ultrafiltration assurant la séparation des métabolites des autres produits du milieu liquide. L'acheminement de ces derniers dans le système 42 est assuré via une conduite d'amenée 44 connectée à la conduite 40 et équipée d'une pompe 46 et d'un réservoir tampon 48. Des réservoirs 50 et 52 assurent la récolte des produits de l'ultrafiltration ; le réservoir 50 la récupération des milieux usés et le réservoir 52 la récupération des métabolites.

Afin de compenser l'extraction en 40 des protéines, lors du prélèvement séquentiel des produits de culture, une conduite d'amenée 54 de solution protéinique dans le réacteur 14 est prévue. Cette conduite est une conduite 54 souple équipée d'une pompe 56 et provient d'un récipient 58 contenant la solution protéinique.

Conformément à l'invention, les conduites 12, 16, 32, 40 et 54 reliant te bioréacteur 14 aux autres éléments du dispositif de culture sont souples afin de ne pas entraver le mouvement de rotation alternatif, de 180° environ, du bioréacteur 14 autour de son axe longitudinal 15 ; ce mouvement de rotation associé à l'inversion du sens de circulation du milieu nourricier dans le bioréacteur 14 évite la sédimention des cellules dans ce dernier.

Bien entendu, lesdites conduites souples peuvent être remplacées par des conduites rigides équipées de raccords coaxiaux ou semi-coaxiaux tournants, étanches et stérilisables.

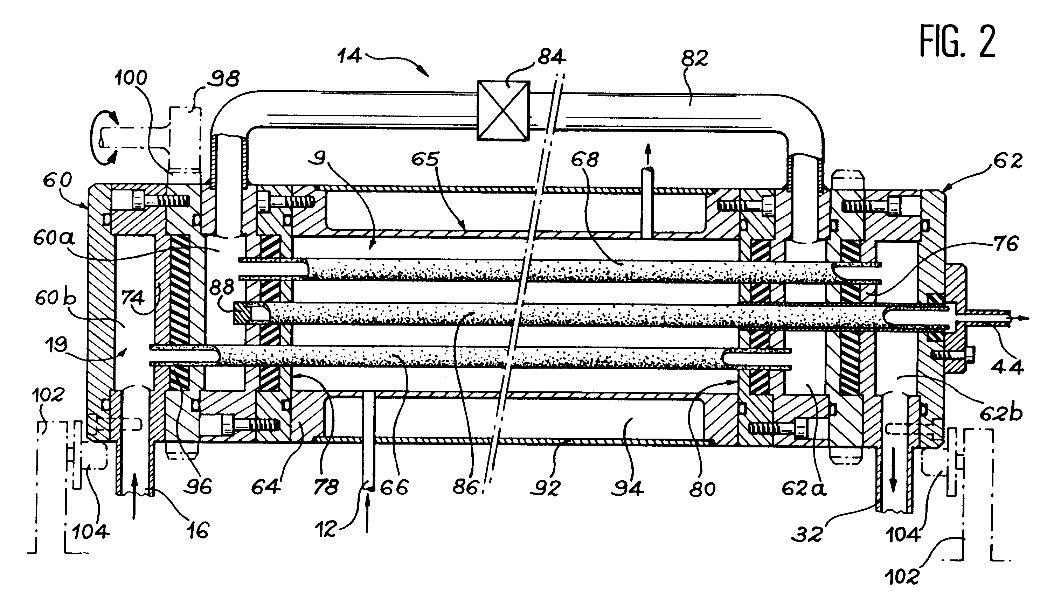

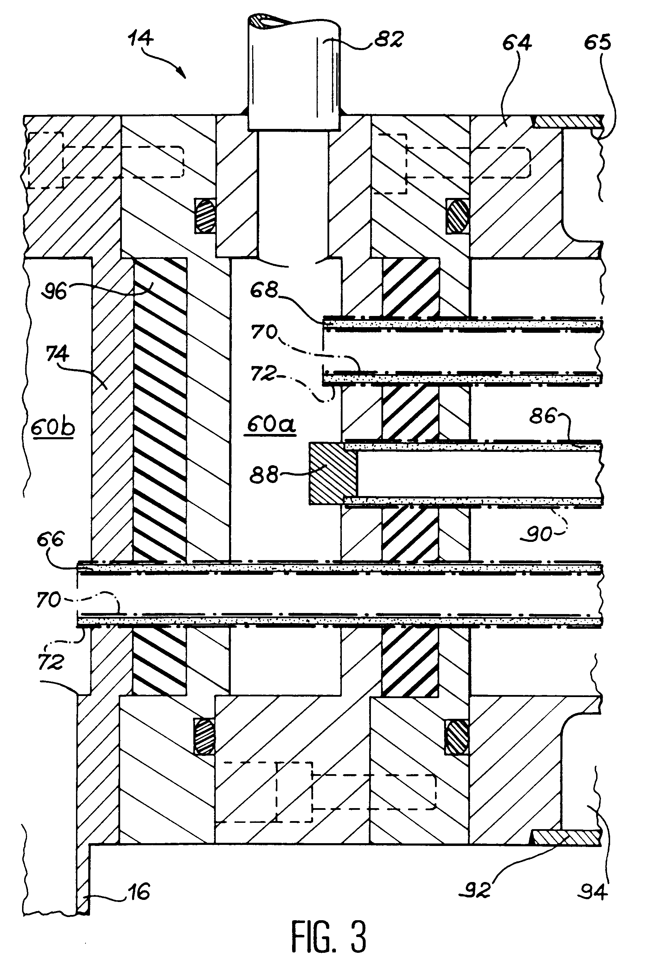

Le bioréacteur 14 selon l'invention, représenté en détail sur les figures 2 et 3, comprend deux enceintes 60 et 62, cylindriques disposées longitudinalement et montées à chaque extrémité d'une virole cylindrique interne 64 destinée à contenir le milieu de culture 9. Des premiers et seconds tubes 66 et 68 minéraux poreux et en particulier en carbone poreux, logés dans la virole 64 et disposés selon l'axe longitudinal du bioréacteur 14, assurent la mise en communication des enceintes 60 et 62.

Ces tubes 66 et 68 sont destinés à la circulation du milieu nourricier provenant du réacteur 18 et sont placés au contact du milieu de culture 9 contenu dans la chambre 65 définie par la virole interne 64 et les parois en regard 78 et 80 des deux enceintes 60 et 62.

Conformément à l'invention, chaque tube 66 et 68 est équipé d'une membrane filtrante poreuse interne 70 et d'une membrane filtrante poreuse externe 72. Ces membranes 70 et 72 sont des membranes minérales microporeuses notamment en zircone.

Les membranes filtrantes intérieures 70 assurent te passage des molécules du milieu nourricier 19 de masse moléculaire moyenne vers le milieu de culture 9 mais empêchent le passage des virus et des produits de corrosion présents et autres particules de masse moléculaire élevée dans le milieu nourricier vers le milieu de culture. Elles constituent donc une barrière contre la pollution. Leur seuil de coupure est choisi en particulier voisin de 0,08 micromètre.

Les membranes filtrantes extérieures 72 laissent passer les molécules du milieu nourricier de faibles masses moléculaires vers le milieu de culture mais leur seuil de coupure est plus bas que celui des membranes intérieures 70. Ces barrières extérieures 72 constituent une barrière efficace à la plupart des protéines et aux cellules contenues dans le milieu de culture 9. Le seuil de coupure de ces membranes externes 72 est en particulier de 10 000 Daltons.

Le fait que te seuil de coupure de la membrane filtrante interne est plus élevé que celui de la membrane externe correspondante permet de ne pas trop diminuer la perméabilité globale de l'ensemble tube-membranes.

Conformément à l'invention, les deux enceintes 60 et 62 sont partagées chacune en deux chambres, une chambre interne et une chambre externe par une cloison perpendiculaire aux tubes 66 et 68. La cloison de la chambre 60 porte la référence 74 et délimite une chambre interne 60a et une chambre externe 60b et l'enceinte 62 est équipée d'une cloison 76 délimitant deux chambres, une chambre interne 62a et une chambre externe 62b.

Le tube 66 traversant tes parois 78 et 80 en regard des deux enceintes 60 et 62 ainsi que la cloison 74 relie la chambre externe 60b de l'enceinte 60 à la chambre interne 62a de la chambre 62. De même, le tube poreux 68 traversant tes parois en regard 78 et 80 des deux enceintes 60 et 62 et la cloison 76 assure la mise en communication de l'enceinte interne 60a avec l'enceinte externe 62b.

Le milieu nourricier 19 riche en éléments nécessaires à la culture des cellules et provenant du réacteur 18 arrive par exemple dans la chambre 60b, traverse la paroi poreuse du tube 66 équipé de ces membranes interne 70 et externe 72 et alimente le milieu de culture 9 des cellules en milieu frais puis débouche dans la chambre interne 62a. Le passage du milieu nourricier à travers la première barrière poreuse (tube 66 + membranes) est assuré grâce à une surpression du milieu nourricier par rapport au milieu de culture.

A cet effet, le bioréacteur 14 est équipé d'une conduite externe 82 mettant en communication la chambre interne 60a avec la chambre interne 62a. Cette conduite 82 est équipée d'une vanne 84 permettant de modifier la perte de charge dans ladite conduite 82 pour augmenter la pression dans les premiers tubes 66. Le liquide en passant dans la seconde barrière tubulaire constituée par le tube 68 et ses membranes interne et externe 70 et 72 se charge en liquide appauvri en éléments nutritifs des cellules et retourne au réacteur 18 en vue de se recharger en éléments nutritifs.

Le réacteur 14 comporte en outre un autre tube minéral poreux 86, notamment en carbone poreux, isolé des deux enceintes 60 et 62, et par conséquent des chambres 60a, 60b, 62a et 62b, destiné à l'extraction des substances ou métabolites produits par les cellules du milieu de culture 9. L'extrémité du tube non destiné à l'extraction des métabolites est équipée à ce sujet d'un bouchon d'étanchéité 88.

Ce tube poreux 86 est équipé d'une membrane filtrante microporeuse externe 90 notamment en zircone permettant le prélèvement des métabolites produits par tes cellules cultivées dans le milieu de culture 9 et entourant les différents tubes 66, 68, 86. Cette membrane 90 présente un seuil de coupure du même ordre de grandeur que celui des membranes filtrantes intérieures 70, soit un seuil de coupure de 0,08 micromètre. Cette membrane microporeuse 90 est imperméable aux cellules, mais perméable aux macromolécules qui peuvent ainsi être soutirées pendant le fonctionnement en continu du bioréacteur.

Le prélèvement du milieu liquide chargé en protéines et en métabolites peut être fait de manière séquentielle dans le volume tampon 48.

Selon l'invention, la virole cylindrique 74 est entourée d'une seconde virole cylindrique 92 externe afin de former un espace annulaire 94 entre les deux viroles dans lequel on fait circuler un liquide assurant la régulation en température du milieu de culture 9.

Des joints d'étanchéité en silicone 96 sont prévus dans les cloisons 74, 76 et les parois 78 et 80 des enceintes respectivement 60 et 62. Les enceintes 60 et 62 ainsi que la virole interne 64 sont de préférence réalisées en acier inoxydable. Ainsi, les cellules animales du milieu de culture sont uniquement en contact avec de l'acier inox, le silicone des joints et le matériau constituant tes barrières minérales (tube + membranes).

La rotation du bioréacteur 14 autour de son axe longitudinal peut être assurée par n'importe quel système hydraulique, pneumatique ou mécanique et par exemple à l'aide d'un pignon 98 agissant sur une couronne dentée 100 montée sur l'enceinte 60. Pour assurer le support du bioréacteur 14 tout en permettant sa rotation, celui-ci est équipé d'un support 102 pourvu d'un système à galet 104.

Le bioréacteur décrit précédemment ne comporte que deux tubes 66 et 68 destinés à la circulation du milieu nourricier et qu'un tube pour l'extraction des métabolites et en particulier des anticorps monoclonaux.

Cette représentation de trois tubes uniquement a été faite en vue d'une simplification. En effet, de façon pratique, on utilise en fait un ensemble de tubes par exemple 5, 7, 19, 37 voire même davantage de tubes disposés régulièrement à l'intérieur du bioréacteur. Afin d'assurer la connexion alternée des chambres internes et externes des deux enceintes 60 et 62, le nombre de tubes 66 et le nombre de tubes 68 doivent être égaux.

L'arrangement le plus favorable à l'homogénéité est un pas triangulaire équilatéral pour ces tubes bien que d'autres arrangements tels qu'un pas carré, pentagonal régulier ou autre puissent être envisagés.

Le bioréacteur de l'invention est d'autant plus performant qu'il comporte un grand nombre de tubes poreux tels que 66 et 68 destinés à l'alimentation en milieu nourricier et à condition que le nombre de tubes d'extraction n'augmente pas trop.

La forme et le mode de fonctionnement du dispositif conforme à l'invention ont été sélectionnés pour qu'une production continue sur une longue période de métabolites soit possible même avec un milieu de culture non exempt de protéines. La limitation du colmatage des barrières poreuses (membranes + tube) est obtenue grâce aux choix judicieux des membranes microporeuses de zircone décrites ci-dessus mais aussi en limitant les pressions de transferts entre les différents tubes.

## Revendications

1. Bioréacteur pour la culture de cellules animales comportant :
- une chambre de culture cellulaire (65), formée par une virole (64) interne et les parois en regard (78, 80) de deux enceintes, une enceinte d'entrée (60) et une enceinte de sortie (62), renfermant à la fois les cellules et le milieu de culture, chaque enceinte étant équipée d'une cloison interne (74, 76) partageant ladite enceinte en une première (60a, 62a) et une seconde (60b, 62b) chambres respectivement intérieure et extérieure ;
- au moins un premier tube minéral poreux (66) reliant la chambre externe (60b) de l'enceinte d'entrée (60) à la chambre interne (62a) de l'enceinte de sortie (62) et traversant ladite chambre de culture (65) pour alimenter le milieu de culture en milieu nourricier, ledit premier tube minéral poreux (66) comportant sur sa face interne une première membrane (70) fillrante microporeuse autorisant le passage des molécules du milieu nourricier (19) de masse moléculaire moyenne, mais arrêtant les particules de masse moléculaire élevée, et sur sa face externe une seconde membrane (72) microfiltrante permettant le passage des molécules de faible masse moléculaire et jouant te rôle de barrière pour les cellules et la plupart des protéines présentes dans la chambre de culture cellulaire ;
- au moins un deuxième tube minéral poreux (68) reliant la chambre interne (60a) de l'enceinte d'entrée (60) à la chambre externe (62b) de l'enceinte de sortie (62) en traversant la chambre (65) de culture cellulaire dont il permet d'extraire les métabolites de faible masse moléculaire, ledit deuxième tube (68) comportant sur ses faces externe et interne des membranes filtrantes (70, 72) identiques à celles du premier tube minéral poreux ;
- une conduite (82) de liaison entre les deux chambres internes (60a, 62a) des enceintes d'entrée et de sortie, dont la perte de charge supplémentaire a pour conséquence un meilleur transfert de matière à travers les premiers (66) et seconds (68) tubes minéraux poreux et qui, en provoquant une circulation de même sens du milieu nourricier à travers lesdits premiers et seconds tubes minéraux poreux, permet d'améliorer l'homogénéité du bioréacteur.

2. Bioréacteur selon la revendication 1, caractérisé en ce que la conduite (82) de liaison entre les deux chambres internes (60a, 62a) est équipée d'une vanne de réglage (84) pour ajuster et contrôler la perte de charge dans ladite conduite (82) et obtenir ainsi un transfert très sensiblement constant sur toute la longueur desdits premiers (66) et seconds (68) tubes minéraux poreux.

3. Bioréacteur selon la revendication 1, caractérisé en ce qu'il comprend au moins un troisième tube (86) minéral poreux isolé par rapport aux deux enceintes (60, 62), traversant ladite chambre de culture (65) et ayant la face extérieure recouverte d'une troisième membrane (90) filtrante microporeuse imperméable aux cellules, mais perméable aux macromolécules produites dans la chambre (65) de culture cellulaire.

4. Bioréacteur selon la revendication 3, caractérisé en ce que les premières (70), secondes (72) et troisième (90) membranes sont des membranes minérales.

5. Bioréacteur selon la revendication 3, caractérisé en ce que les premières, secondes et troisième membranes (70, 72, 90) sont constituées en un matériau choisi parmi Al₂O₃, TiO₂ et ZrO₂.

6. Bioréacteur selon la revendication 1, caractérisé en ce que les secondes membranes filtrantes (72) ont un seuil de coupure inférieur à celui des premières membranes filtrantes (70).

7. Bioréacteur selon la revendication 1, caractérisé en ce que les premiers et seconds tubes (66, 68, 86) sont en carbone poreux.

8. Bioréacteur selon la revendication 3, caractérisé en ce que la troisième membrane filtrante (90) a un seuil de coupure voisin de celui des premières membranes (70).

9. Bioréacteur selon la revendication 1, caractérisé en ce qu'une virole externe (92) entourant la virole interne est prévue pour former un espace annulaire (94) destiné à la circulation d'un fluide de régulation en température du milieu de culture.

10. Bioréacteur selon la revendication 1, caractérisé en ce que des moyens (34) sont prévus pour inverser périodiquement le sens de circulation du milieu nourricier (19) dans les premiers tubes (66, 68).

11. Bioréacteur selon la revendication 1, caractérisé en ce qu'il comprend des moyens (98, 100) pour le faire tourner alternativement de 180°C environ autour d'un axe longitudinal (15).

12. Dispositif pour la culture en continu de cellules animales comprenant un réacteur (18) de contrôle et de préparation du milieu nourricier, connecté à un bioréacteur (14) selon la revendication 1, via des conduites souples (16, 32), et des moyens (48, 46, 42, 52) pour extraire en continu ou séquentiellement les substances produites par les cellules, ces moyens étant connectés au bioréacteur (14) via des conduites souples (40).

## Claims

1. Bioreactor for the culture of animal cells comprising: a cellular culture chamber (65), formed by a sleeve (64) and facing walls (78,80) of two enclosures, respectively the intake enclosure (60) and the discharge enclosure (62), containing on each occasion the cells and the culture medium, each enclosure being provided with an internal partition (74,76) subdividing said enclosure into a first (60a, 62a) and a second (60b,62b) respectively internal and external chambers;
at least one first porous mineral tube (66) connecting the external chamber (60b) of the intake enclosure (60) to the internal chamber (62a) of the discharge enclosure (62) and traversing said culture chamber (65) for supplying nutrient medium to the culture medium, said first porous mineral tube (66) having on its inner face a first micro-porous filtering membrane (70) allowing the passage of molecules of the nutrient medium (19) with an average molecular weight, but stopping the macromolecules of a high molecular weight, and on its outer face a second micro-filtering membrane (72) permitting the passage of the low molecular weight molecules and serving as a barrier for the cells and most of the proteins present in the cellular culture chamber;
at least one second porous mineral tube (68) connecting the internal chamber (60a) of the intake enclosure (60) to the external chamber (62b) of the discharge enclosure (62), whilst traversing the cellular culture chamber (65) from which it makes it possible to extract the low molecular weight metabolites, said second tube (68) having on its outer and inner faces filtering membranes (70,72) identical to those of the first porous mineral tube;
a connecting pipe (82) between the two internal chambers (60a,62a) of the intake and discharge enclosures, whose supplementary pressure drop has the effect of bringing about a better material transfer through the first (66) and second (68) porous mineral tubes and which, by bringing about a flow in the same direction of the nutrient medium through said first and second porous mineral tubes, makes it possible to improve the homogeneity of the bioreactor.

2. Bioreactor according to claim 1, characterized in that the connecting pipe (82) between the two internal chambers (60a,62a) is equipped with a regulating valve (84) for adjusting and checking the pressure drop in said pipe (82) and for thus obtaining a very substantially constant transfer over the entire length of said first (66) and second (68) porous mineral tubes.

3. Bioreactor according to claim 1, characterized in that it comprises at least one third porous mineral tube (86) isolated from the two enclosures (60,62), whilst traversing said culture chamber (65) and having its outer face covered by a third microporous filtering membrane (90), which is impermeable to the cells, but permeable to the macromolecules produced in the cellular culture chamber (65).

4. Bioreactor according to claim 3, characterized in that the first (70), second (72) and third (90) membranes are mineral membranes.

5. Bioreactor according to claim 3, characterized in that the first, second and third membranes (70,72,90) are made from a material chosen from among Al₂O₃, TiO₂ and ZrO₂.

6. Bioreactor according to claim 1, characterized in that the second filtering membranes (72) have a cutoff threshold below that of the first filtering membranes (70).

7. Bioreactor according to claim 1, characterized in that the first and second tubes (66,68,86) are made from porous carbon.

8. Bioreactor according to claim 3, characterized in that the third filtering membrane (90) has a cutoff threshold close to that of the first membrane (70).

9. Bioreactor according to claim 1, characterized in that an outer sleeve (92) surrounding the inner sleeve is provided for forming an annular space (94) for the circulation of a fluid for regulating the temperature of the culture medium.

10. Bioreactor according to claim 1, characterized in that means (34) are provided for periodically reversing the flow direction of the nutrient medium (19) in the first tubes (66,68).

11. Bioreactor according to claim 1, characterized in that it comprises means (98,100) for bringing about an alternate rotation thereof by approximately 180^{o} about a longitudinal axis (15).

12. Apparatus for the continuous culture of animal cells comprising a reactor (18) for checking and preparing the nutrient medium and connected to a bioreactor (14) in accordance with claim 1, via flexible pipes (16,32) and means (48,46, 42,52) for the continuous or sequential extraction of the substances produced by the cells, said means being connected to the bioreactor (14) via flexible pipes (40).

## Patentansprüche

1. Bioreaktor zur Kultivierung von tierischen Zellen, enthaltend :
- eine Zellkulturkammer (65), gebildet durch einen Innenring (64) und den sich gegenüberstehenden Wänden (78, 80) von zwei Räumen, einem Eintrittsraum (60) und einem Austrittsraum (62), die zugleich die Zellen und den Nährboden umschließen, wobei jeder Raum ausgestattet ist mit einer Innentrennwand (74, 76), den genannten Raum trennend in eine erste (60a, 62a) und eine zweite (60b, 62b) Innen- beziehungsweise Außenkammer;
- wenigstens ein erstes poröses Mineralrohr (66), das die Außenkammer (60b) des Eintrittsraums (60) mit der Innenkammer (62a) des Austrittsraums (62) verbindet und die genannte Kulturkammer (65) durchquert, um den Nährboden mit Nährmittel zu versorgen, wobei das genannte erste poröse Mineralrohr (66) an seiner Innenfläche eine erste mikroporöse Filtriermembran (70) enthält, die den Durchgang der Moleküle mittlerer molarer Masse des Nährmittels (19) zuläßt, aber die Partikel großer molarer Masse zurückhält, und auf seiner Außenfläche eine zweite, mikrofiltrierende Membran (72), welche den Durchgang der Molküle kleiner molarer Masse zuläßt und die Rolle einer Barriere spielt für die Zellen und meisten Proteine, die in der Zellkulturkammer vorhanden sind;
- wenigstens ein zweites poröses Mineralrohr (68), das die Innenkammer (60a) des Eintrittsraums (60) mit der Außenkammer (62b) des Austrittsraums (62) verbindet, indem es die Zellkulturkammer durchquert und somit ermöglicht, ihr die Metaboliten geringer molarer Masse zu entziehen, wobei das genannte zweite Rohr (68) an seinen Flächen, außen und innen, Filtriermembranen (70, 72) aufweist, identisch mit denen des ersten porösen Mineralrohrs;
- eine Verbindungsleitung (82) zwischen den beiden Innenkammern (60a, 62a) der Eintritts- und Austrittsräume, deren zusätzlicher Druckverlust einen besseren Stoffluß durch die ersten (66) und zweiten (68) porösen Mineralrohre zur Folge hat und die, mittels Erzeugung einer gleichgerichteten Zirkulation des Nährmittels durch die ersten und zweiten porösen Mineralrohre eine Verbesserung der Homogenität des Bioreaktors gewährleistet.

2. Bioreaktor nach Anspruch 1, dadurch gekennzeichnet, daß die Verbindungsleitung (82) zwischen den beiden Innenkammern (60a, 62a) ausgestattet ist mit einem Regelventil (84), um den Druckverlust in der Leitung (82) anzupassen und zu steuern und somit einen im wesentlichen konstanten Fluß über die gesamte Länge der genannten ersten (66) und zweiten (68) porösen Mineralrohre zu erhalten.

3. Bioreaktor nach Anspruch 1, dadurch gekennzeichnet, daß er wenigstens ein drittes poröses Mineralrohr (86) enthält, isoliert bezüglich der beiden Räume (60, 62), das die genannte Kulturkammer (65) durchquert und dessen Außenseite mit einer dritten, mikroporösen Filtriermembran (90) überzogen ist, undurchlässig für die Zellen, aber durchlässig für die in der Zellkulturkammer (65) produzierten Makromoleküle.

4. Bioreaktor nach Anspruch 3, dadurch gekennzeichnet, daß die ersten (70), zweiten (72) und dritten (90) Membranen mineralische Membranen sind.

5. Bioreaktor nach Anspruch 3, dadurch gekennzeichnet, daß die ersten, zweiten und dritten Membranen (70, 72, 90) aus einem Material bestehen, das ausgewählt wird aus Al₂O₃, TiO₂ und ZrO₂.

6. Bioreaktor nach Anspruch 1, dadurch gekennzeichnet, daß die zweiten Filtriermembranen (72) eine Trennschwelle haben, die niedriger ist als die der ersten Filtriermembranen (70).

7. Bioreaktor nach Anspruch 1, dadurch gekennzeichnet, daß die ersten und zweiten Rohre (66, 68, 86) aus porösem Karbon sind.

8. Bioreaktor nach Anspruch 3, dadurch gekennzeichnet, daß die dritte Filtriermembran (90) eine Trennschwelle hat, die der der ersten Membranen (70) nahekommt.

9. Bioreaktor nach Anspruch 1, dadurch gekennzeichnet, daß ein den Innenring umgebender Außenring (92) vorgesehen ist, um einen ringförmigen Raum (94) zu schaffen, bestimmt für den Durchfluß eines Fluids zur Temperaturregelung des Nährbodens.

10. Bioreaktor nach Anspruch 1, dadurch gekennzeichnet, daß die Einrichtungen (34) vorgesehen sind, um periodisch die Flußrichtung des Nährmittels (19) in den ersten Rohren (66,68) umzukehren.

11. Bioreaktor nach Anspruch 1, dadurch gekennzeichnet, daß er Einrichtungen (98, 100) umfaßt, um ihn wechselweise um ungefähr 180° um eine Längsachse (15) herum zu drehen.

12. Vorrichtung zur kontinuierlichen Kultivierung von tierischen Zellen, umfassend einen Reaktor (18) zur Kontrolle und Vorbereitung des Nährmittels, angeschlossen an einen Bioreaktor (14) gemäß Anspruch 1, über biegsame Leitungen (16, 32), und Einrichtungen (48, 46, 42, 52), um kontinuierlich oder sequentiell die durch die Zellen produzierten Substanzen zu extrahieren, wobei diese Einrichtungen mit dem Bioreaktor (14) über biegsame Leitungen (40) verbunden sind.
